(19) Europäisches Patentamt
European Patent Office
Office européen des brevets

(11) EP 3 450 551 A1

(12) EUROPEAN PATENT APPLICATION

(43) Date of publication:
**06.03.2019 Bulletin 2019/10**

(51) Int Cl.:
***C12N 9/14*** *(2006.01)*

(21) Application number: **18192063.8**

(22) Date of filing: **31.08.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.09.2017 KR 20170111925**

(71) Applicant: **Samsung Electronics Co., Ltd.**
**Gyeonggi-do 16677 (KR)**

(72) Inventors:
- **JUNG, Yukyung**
  **16678 Suwon-si, Gyeonggi-do (KR)**
- **KIM, Taeyong**
  **16678 Suwon-si, Gyeonggi-do (KR)**
- **BYUN, Jongwon**
  **16678 Suwon-si, Gyeonggi-do (KR)**
- **PARK, Jinhwan**
  **16678 Suwon-si, Gyeonggi-do (KR)**

(74) Representative: **Grünecker Patent- und Rechtsanwälte PartG mbB**
**Leopoldstraße 4**
**80802 München (DE)**

(54) **HALOACID DEHALOGENASE SUPERFAMILY PROTEIN VARIANT AND METHOD OF REDUCING THE CONCENTRATION OF A FLUORINE-CONTAINING COMPOUND IN A SAMPLE USING THE SAME**

(57) Provided are a haloacid dehalogenase superfamily protein variant and a method of reducing a concentration of a fluorine-containing compound in sample using the same.

FIG. 1

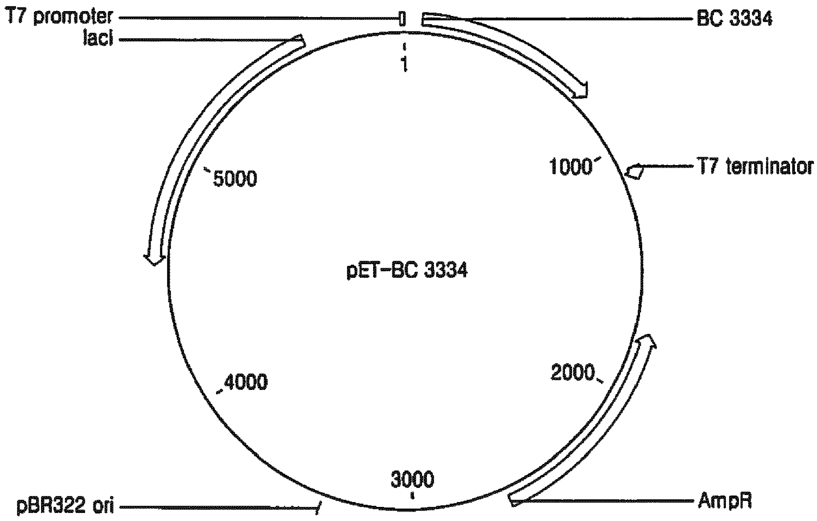


EP 3 450 551 A1

<110>    Samsung Electronics Co., Ltd.

<120>    Haloacid dehalogenase superfamily protein variant and method of reducing the concentration of a fluorine-containing compound in a sample
using the same

<130>    EP119011FZ358pau

<140>    not yet assigned
<141>    herewith

<150>    KR10-2017-0111925
<151>    2017-09-01

<160>    33

<170>    KopatentIn 2.0

<210>    1
<211>    236
<212>    PRT
<213>    Bacillus cereus

<400>    1

```
Met Lys Tyr Lys Val Ile Leu Phe Asp Val Asp Asp Thr Leu Leu Asp
 1               5                  10                  15

Phe Pro Glu Thr Glu Arg His Ala Leu His Asn Ala Phe Val Gln Phe
            20                  25                  30

Asp Met Pro Thr Gly Tyr Asn Asp Tyr Leu Ala Ser Tyr Lys Glu Ile
        35                  40                  45

Ser Asn Gly Leu Trp Arg Asp Leu Glu Asn Lys Met Ile Thr Leu Ser
    50                  55                  60

Glu Leu Ala Val Asp Arg Phe Arg Gln Leu Phe Ala Leu His Asn Ile
65                  70                  75                  80

Asp Val Asp Ala Gln Gln Phe Ser Asp Val Tyr Leu Glu Asn Leu Gly
                85                  90                  95

Lys Glu Val His Leu Ile Glu Gly Ala Val Gln Leu Cys Glu Asn Leu
            100                 105                 110

Gln Asp Cys Lys Leu Gly Ile Ile Thr Asn Gly Tyr Thr Lys Val Gln
        115                 120                 125

Gln Ser Arg Ile Gly Asn Ser Pro Leu Cys Asn Phe Phe Asp His Ile
    130                 135                 140

Ile Ile Ser Glu Glu Val Gly His Gln Lys Pro Ala Arg Glu Ile Phe
145                 150                 155                 160

Asp Tyr Ala Phe Glu Lys Phe Gly Ile Thr Asp Lys Ser Ser Val Leu
                165                 170                 175

Met Val Gly Asp Ser Leu Thr Ser Asp Met Lys Gly Gly Glu Asp Tyr
            180                 185                 190

Gly Ile Asp Thr Cys Trp Tyr Asn Pro Ser Leu Lys Glu Asn Gly Thr
        195                 200                 205
```

```
Asp Val Asn Pro Thr Tyr Glu Val Glu Ser Leu Leu Gln Ile Leu Glu
    210                 215                 220

Ile Val Glu Val Ala Glu Glu Lys Val Ala Ser Phe
225                 230                 235


<210>   2
<211>   711
<212>   DNA
<213>   Bacillus cereus

<400>   2
atgaaataca aagttatatt attcgacgta gatgatacat tattagattt ccctgaaacg      60

gaaagacacg cattacataa tgcgtttgta cagtttgata tgcctacagg gtataatgat     120

tatcttgcaa gctataaaga gattagtaat ggattatgga gagatttaga aaataaaatg     180

attacgctaa gtgaattagc agtagatcga tttagacaat tatttgcact tcataatata     240

gacgtagatg cacagcaatt tagtgatgta taccttgaaa atttagggaa ggaagtacat     300

cttatagaag gcgcagtaca attatgtgaa aatctacaag attgcaagtt aggtattatt     360

acgaatggat atacgaaggt gcaacaatca agaatcggaa attcaccttt atgtaatttc     420

tttgatcaca ttattatttc tgaagaagtt ggtcatcaaa aaccagcacg tgagattttt     480

gattatgcgt ttgagaagtt tgggattact gataaatcaa gcgtactaat ggttggagat     540

tcgttaactt ctgatatgaa aggcggagaa gattacggca ttgatacgtg ttggtataat     600

ccgagtttga aagaaaacgg gacagatgtt aacccgactt atgaagtgga gagtctgctc     660

caaattttag aaattgtaga agtggcggaa gaaaaggtag cttcatttta a             711


<210>   3
<211>   40
<212>   DNA
<213>   Artificial Sequence

<220>
<223>   primer


<400>   3
aagaaggaga tataccatga aatacaaagt tatattattc                            40


<210>   4
<211>   41
<212>   DNA
<213>   Artificial Sequence

<220>
<223>   primer


<400>   4
gcattatgcg gccgcaagct ttaaaatgaa gctacctttt c                          41
```

<210> 5
<211> 236
<212> PRT
<213> Bacillus bombysepticus SF3

<400> 5
```
Met Lys Tyr Lys Phe Ile Leu Phe Asp Val Asp Asp Thr Leu Leu Asp
1               5                   10                  15

Phe Pro Glu Thr Glu Arg His Ala Leu His Asn Ala Phe Val Gln Phe
            20                  25                  30

Gly Met Pro Thr Gly Tyr Asn Asp Tyr Leu Ala Ser Tyr Lys Glu Ile
        35                  40                  45

Ser Asn Gly Leu Trp Arg Asp Leu Glu Asn Lys Met Ile Thr Leu Ser
    50                  55                  60

Glu Leu Ala Val Asp Arg Phe Arg Gln Leu Phe Ala Leu His Asn Ile
65                  70                  75                  80

Lys Val Asp Ala Gln Gln Phe Ser Asp Val Tyr Leu Glu Asn Leu Gly
                85                  90                  95

Lys Glu Val His Leu Ile Glu Gly Ala Val Gln Leu Cys Glu Asp Leu
            100                 105                 110

Gln Asp Cys Lys Leu Gly Ile Ile Thr Asn Gly Tyr Thr Lys Val Gln
        115                 120                 125

Gln Ser Arg Ile Gly Asn Ser Pro Val Cys Asn Phe Phe Asp His Ile
    130                 135                 140

Ile Ile Ser Glu Glu Val Gly His Gln Lys Pro Ala Arg Glu Ile Phe
145                 150                 155                 160

Asp Tyr Ala Phe Glu Lys Phe Gly Ile Thr Asp Lys Ser Ser Val Leu
                165                 170                 175

Met Val Gly Asp Ser Leu Ser Ser Asp Met Arg Gly Gly Glu Asp Tyr
            180                 185                 190

Gly Ile Asp Thr Cys Trp Tyr Asn Pro Ser Leu Lys Glu Asn Arg Thr
        195                 200                 205

Asp Val Lys Pro Ser Tyr Glu Val Glu Ser Leu Leu Gln Ile Leu Glu
    210                 215                 220

Ile Val Glu Val Thr Lys Glu Lys Val Ala Ser Phe
225                 230                 235
```

<210> 6
<211> 711
<212> DNA
<213> Bacillus bombysepticus SF3

<400> 6
```
atgaaataca aatttatatt attcgacgta gacgatacat tattagattt ccctgaaacg      60

gaaagacacg cattacataa tgcgtttgta cagttcggga tgcctacagg gtataatgat     120
```

tatcttgcaa gttataaaga gattagtaat ggattatgga gagatttaga aaataaaatg 180

attacgctaa gtgaattagc ggtagatcga tttagacaat tatttgccct tcataatata 240

aaagtagatg cgcagcaatt tagcgatgta tatcttgaaa acttagggaa agaagtacat 300

cttatagaag gtgcagtgca attatgtgag gatctacaag attgcaagtt aggtattatt 360

acgaatggat atacgaaggt gcaacaatcg agaattggaa attcgcctgt atgtaatttc 420

tttgatcata ttattatttc agaagaggtt ggtcatcaaa aaccagcacg tgagattttt 480

gattatgcgt ttgaaaagtt tgggattaca gataaatcaa gtgtattaat ggttggagat 540

tcgctttctt ctgatatgag aggcggagaa gattacggca ttgatacgtg ttggtataat 600

ccgagtttga aagaaaatag gacagatgtt aagccgtctt atgaagtgga gagtctgcta 660

caaattttag aaattgtaga agtgactaaa gaaaaagtag cttcatttta a 711

<210> 7
<211> 37
<212> DNA
<213> Artificial Sequence

<220>
<223> primer

<400> 7
aagaaggaga tataccatga aatacaaatt tatatta 37

<210> 8
<211> 39
<212> DNA
<213> Artificial Sequence

<220>
<223> primer

<400> 8
ggtggtggtg gtgctcgatt aaaatgaagc tactttttc 39

<210> 9
<211> 39
<212> DNA
<213> Artificial Sequence

<220>
<223> primer

<400> 9
aaactgggca ttatcaccta tggttatacc aaagtgcag 39

<210> 10
<211> 39

<212> DNA
<213> Artificial Sequence

<220>
<223> primer

<400> 10
ctgcactttg gtataaccat aggtgataat gcccagttt 39

<210> 11
<211> 39
<212> DNA
<213> Artificial Sequence

<220>
<223> primer

<400> 11
aaactgggca ttatcaccgt gggttatacc aaagtgcag 39

<210> 12
<211> 39
<212> DNA
<213> Artificial Sequence

<220>
<223> primer

<400> 12
ctgcactttg gtataaccca cggtgataat gcccagttt 39

<210> 13
<211> 39
<212> DNA
<213> Artificial Sequence

<220>
<223> primer

<400> 13
gttggagatt cgctttctca tgatatgaga ggcggagaa 39

<210> 14
<211> 39
<212> DNA
<213> Artificial Sequence

<220>
<223> primer

<400> 14
ttctccgcct ctcatatcat gagaaagcga atctccaac 39

<210> 15
<211> 39
<212> DNA
<213> Artificial Sequence

<220>
<223> primer

<400> 15
gttggagatt cgctttctca ggatatgaga ggcggagaa 39

<210> 16
<211> 39
<212> DNA
<213> Artificial Sequence

<220>
<223> primer

<400> 16
ttctccgcct ctcatatcct gagaaagcga atctccaac 39

<210> 17
<211> 39
<212> DNA
<213> Artificial Sequence

<220>
<223> primer

<400> 17
gttggagatt cgctttctga tgatatgaga ggcggagaa 39

<210> 18
<211> 39
<212> DNA
<213> Artificial Sequence

<220>
<223> primer

<400> 18
ttctccgcct ctcatatcat cagaaagcga atctccaac 39

<210> 19
<211> 236
<212> PRT
<213> Artificial Sequence

<220>
<223> BC3334 N122Y mutant

<400> 19
Met Lys Tyr Lys Val Ile Leu Phe Asp Val Asp Asp Thr Leu Leu Asp

```
1               5                   10                  15

Phe Pro Glu Thr Glu Arg His Ala Leu His Asn Ala Phe Val Gln Phe
            20                  25                  30

Asp Met Pro Thr Gly Tyr Asn Asp Tyr Leu Ala Ser Tyr Lys Glu Ile
        35                  40                  45

Ser Asn Gly Leu Trp Arg Asp Leu Glu Asn Lys Met Ile Thr Leu Ser
    50                  55                  60

Glu Leu Ala Val Asp Arg Phe Arg Gln Leu Phe Ala Leu His Asn Ile
65                  70                  75                  80

Asp Val Asp Ala Gln Gln Phe Ser Asp Val Tyr Leu Glu Asn Leu Gly
                85                  90                  95

Lys Glu Val His Leu Ile Glu Gly Ala Val Gln Leu Cys Glu Asn Leu
            100                 105                 110

Gln Asp Cys Lys Leu Gly Ile Ile Thr Tyr Gly Tyr Thr Lys Val Gln
        115                 120                 125

Gln Ser Arg Ile Gly Asn Ser Pro Leu Cys Asn Phe Phe Asp His Ile
    130                 135                 140

Ile Ile Ser Glu Glu Val Gly His Gln Lys Pro Ala Arg Glu Ile Phe
145                 150                 155                 160

Asp Tyr Ala Phe Glu Lys Phe Gly Ile Thr Asp Lys Ser Ser Val Leu
                165                 170                 175

Met Val Gly Asp Ser Leu Thr Ser Asp Met Lys Gly Gly Glu Asp Tyr
            180                 185                 190

Gly Ile Asp Thr Cys Trp Tyr Asn Pro Ser Leu Lys Glu Asn Gly Thr
        195                 200                 205

Asp Val Asn Pro Thr Tyr Glu Val Glu Ser Leu Leu Gln Ile Leu Glu
    210                 215                 220

Ile Val Glu Val Ala Glu Glu Lys Val Ala Ser Phe
225                 230                 235
```

```
<210>   20
<211>   711
<212>   DNA
<213>   Artificial Sequence

<220>
<223>   BC3334 N122Y mutant


<400>   20
atgaaatata aagttattct gtttgacgtt gatgacaccc tgctggattt cccggaaacc      60

gaacgtcacg cgctgcataa cgcctttgtt cagttcgata tgccgacggg ttacaacgac     120

tatctggcgt cttacaaaga aatctccaac ggcctgtggc gtgatctgga aaacaaaatg     180

atcactctgt ctgaactggc tgttgaccgt ttccgccagc tgttcgctct gcacaacatc     240
```

gacgtggacg cgcagcagtt ttctgatgtg tacctggaga acctgggtaa agaggttcac 300

ctgatcgaag gcgcagtaca actgtgtgaa aatttgcagg actgcaaact gggcattatc 360

acctatggtt ataccaaagt gcagcagtcc cgtatcggca acagcccgct gtgcaacttc 420

tttgatcaca tcatcatcag cgaagaagtc ggtcaccaga aaccggcgcg tgaaatcttc 480

gactacgcat tcgaaaaatt cggtatcacc gataaatcca gcgtgctgat ggtgggtgac 540

tctctgacca gcgatatgaa aggcggcgaa gattacggta ttgatacctg ctggtacaat 600

ccgagcctga aagaaaatgg caccgacgtt aacccgacct acgaagtgga aagcctgctg 660

cagatcctgg aaattgttga agttgctgaa gagaaagtcg cttccttcta a 711

<210> 21
<211> 236
<212> PRT
<213> Artificial Sequence

<220>
<223> BC3334 N122V mutant

<400> 21

Met Lys Tyr Lys Val Ile Leu Phe Asp Val Asp Asp Thr Leu Leu Asp
1 5 10 15

Phe Pro Glu Thr Glu Arg His Ala Leu His Asn Ala Phe Val Gln Phe
20 25 30

Asp Met Pro Thr Gly Tyr Asn Asp Tyr Leu Ala Ser Tyr Lys Glu Ile
35 40 45

Ser Asn Gly Leu Trp Arg Asp Leu Glu Asn Lys Met Ile Thr Leu Ser
50 55 60

Glu Leu Ala Val Asp Arg Phe Arg Gln Leu Phe Ala Leu His Asn Ile
65 70 75 80

Asp Val Asp Ala Gln Gln Phe Ser Asp Val Tyr Leu Glu Asn Leu Gly
85 90 95

Lys Glu Val His Leu Ile Glu Gly Ala Val Gln Leu Cys Glu Asn Leu
100 105 110

Gln Asp Cys Lys Leu Gly Ile Ile Thr Val Gly Tyr Thr Lys Val Gln
115 120 125

Gln Ser Arg Ile Gly Asn Ser Pro Leu Cys Asn Phe Phe Asp His Ile
130 135 140

Ile Ile Ser Glu Glu Val Gly His Gln Lys Pro Ala Arg Glu Ile Phe
145 150 155 160

Asp Tyr Ala Phe Glu Lys Phe Gly Ile Thr Asp Lys Ser Ser Val Leu
165 170 175

Met Val Gly Asp Ser Leu Thr Ser Asp Met Lys Gly Gly Glu Asp Tyr
180 185 190

```
Gly Ile Asp Thr Cys Trp Tyr Asn Pro Ser Leu Lys Glu Asn Gly Thr
        195                 200                 205

Asp Val Asn Pro Thr Tyr Glu Val Glu Ser Leu Leu Gln Ile Leu Glu
    210                 215                 220

Ile Val Glu Val Ala Glu Glu Lys Val Ala Ser Phe
225                 230                 235
```

<210> 22
<211> 711
<212> DNA
<213> Artificial Sequence

<220>
<223> BC3334 N122V mutant

<400> 22

```
atgaaatata aagttattct gtttgacgtt gatgacaccc tgctggattt cccggaaacc        60

gaacgtcacg cgctgcataa cgcctttgtt cagttcgata tgccgacggg ttacaacgac       120

tatctggcgt cttacaaaga aatctccaac ggcctgtggc gtgatctgga aaacaaaatg       180

atcactctgt ctgaactggc tgttgaccgt ttccgccagc tgttcgctct gcacaacatc       240

gacgtggacg cgcagcagtt ttctgatgtg tacctggaga acctgggtaa agaggttcac       300

ctgatcgaag gcgcagtaca actgtgtgaa aatttgcagg actgcaaact gggcattatc       360

accgtgggtt ataccaaagt gcagcagtcc cgtatcggca acagcccgct gtgcaacttc       420

tttgatcaca tcatcatcag cgaagaagtc ggtcaccaga aaccggcgcg tgaaatcttc       480

gactacgcat tcgaaaaatt cggtatcacc gataaatcca gcgtgctgat ggtgggtgac       540

tctctgacca gcgatatgaa aggcggcgaa gattacggta ttgatacctg ctggtacaat       600

ccgagcctga aagaaaatgg caccgacgtt aacccgacct acgaagtgga aagcctgctg       660

cagatcctgg aaattgttga agttgctgaa gagaaagtcg cttccttcta a                711
```

<210> 23
<211> 236
<212> PRT
<213> Artificial Sequence

<220>
<223> SF0757 S184H mutant

<400> 23

```
Met Lys Tyr Lys Phe Ile Leu Phe Asp Val Asp Asp Thr Leu Leu Asp
  1               5                  10                  15

Phe Pro Glu Thr Glu Arg His Ala Leu His Asn Ala Phe Val Gln Phe
             20                  25                  30

Gly Met Pro Thr Gly Tyr Asn Asp Tyr Leu Ala Ser Tyr Lys Glu Ile
         35                  40                  45
```

Ser Asn Gly Leu Trp Arg Asp Leu Glu Asn Lys Met Ile Thr Leu Ser
50 55 60

Glu Leu Ala Val Asp Arg Phe Arg Gln Leu Phe Ala Leu His Asn Ile
65 70 75 80

Lys Val Asp Ala Gln Gln Phe Ser Asp Val Tyr Leu Glu Asn Leu Gly
85 90 95

Lys Glu Val His Leu Ile Glu Gly Ala Val Gln Leu Cys Glu Asp Leu
100 105 110

Gln Asp Cys Lys Leu Gly Ile Ile Thr Asn Gly Tyr Thr Lys Val Gln
115 120 125

Gln Ser Arg Ile Gly Asn Ser Pro Val Cys Asn Phe Phe Asp His Ile
130 135 140

Ile Ile Ser Glu Glu Val Gly His Gln Lys Pro Ala Arg Glu Ile Phe
145 150 155 160

Asp Tyr Ala Phe Glu Lys Phe Gly Ile Thr Asp Lys Ser Ser Val Leu
165 170 175

Met Val Gly Asp Ser Leu Ser His Asp Met Arg Gly Gly Glu Asp Tyr
180 185 190

Gly Ile Asp Thr Cys Trp Tyr Asn Pro Ser Leu Lys Glu Asn Arg Thr
195 200 205

Asp Val Lys Pro Ser Tyr Glu Val Glu Ser Leu Leu Gln Ile Leu Glu
210 215 220

Ile Val Glu Val Thr Lys Glu Lys Val Ala Ser Phe
225 230 235

<210> 24
<211> 711
<212> DNA
<213> Artificial Sequence

<220>
<223> SF0757 S184H mutant

<400> 24
atgaaataca aatttatatt attcgacgta gacgatacat tattagattt ccctgaaacg 60

gaaagacacg cattacataa tgcgtttgta cagttcggga tgcctacagg gtataatgat 120

tatcttgcaa gttataaaga gattagtaat ggattatgga gagatttaga aaataaaatg 180

attacgctaa gtgaattagc ggtagatcga tttagacaat tatttgccct tcataatata 240

aaagtagatg cgcagcaatt tagcgatgta tatcttgaaa acttagggaa agaagtacat 300

cttatagaag gtgcagtgca attatgtgag gatctacaag attgcaagtt aggtattatt 360

acgaatggat atacgaaggt gcaacaatcg agaattggaa attcgcctgt atgtaatttc 420

tttgatcata ttattatttc agaagaggtt ggtcatcaaa aaccagcacg tgagattttt 480

gattatgcgt ttgaaaagtt tgggattaca gataaatcaa gtgtattaat ggttggagat 540

tcgctttctc atgatatgag aggcggagaa gattacggca ttgatacgtg ttggtataat 600

ccgagtttga aagaaaatag gacagatgtt aagccgtctt atgaagtgga gagtctgcta 660

caaattttag aaattgtaga agtgactaaa gaaaaagtag cttcatttta a 711

<210> 25
<211> 236
<212> PRT
<213> Artificial Sequence

<220>
<223> SF0757 S184Q mutant

<400> 25

```
Met Lys Tyr Lys Phe Ile Leu Phe Asp Val Asp Asp Thr Leu Leu Asp
1               5                   10                  15

Phe Pro Glu Thr Glu Arg His Ala Leu His Asn Ala Phe Val Gln Phe
            20                  25                  30

Gly Met Pro Thr Gly Tyr Asn Asp Tyr Leu Ala Ser Tyr Lys Glu Ile
        35                  40                  45

Ser Asn Gly Leu Trp Arg Asp Leu Glu Asn Lys Met Ile Thr Leu Ser
    50                  55                  60

Glu Leu Ala Val Asp Arg Phe Arg Gln Leu Phe Ala Leu His Asn Ile
65                  70                  75                  80

Lys Val Asp Ala Gln Gln Phe Ser Asp Val Tyr Leu Glu Asn Leu Gly
                85                  90                  95

Lys Glu Val His Leu Ile Glu Gly Ala Val Gln Leu Cys Glu Asp Leu
            100                 105                 110

Gln Asp Cys Lys Leu Gly Ile Ile Thr Asn Gly Tyr Thr Lys Val Gln
        115                 120                 125

Gln Ser Arg Ile Gly Asn Ser Pro Val Cys Asn Phe Phe Asp His Ile
    130                 135                 140

Ile Ile Ser Glu Glu Val Gly His Gln Lys Pro Ala Arg Glu Ile Phe
145                 150                 155                 160

Asp Tyr Ala Phe Glu Lys Phe Gly Ile Thr Asp Lys Ser Ser Val Leu
                165                 170                 175

Met Val Gly Asp Ser Leu Ser Gln Asp Met Arg Gly Gly Glu Asp Tyr
            180                 185                 190

Gly Ile Asp Thr Cys Trp Tyr Asn Pro Ser Leu Lys Glu Asn Arg Thr
        195                 200                 205

Asp Val Lys Pro Ser Tyr Glu Val Glu Ser Leu Leu Gln Ile Leu Glu
    210                 215                 220

Ile Val Glu Val Thr Lys Glu Lys Val Ala Ser Phe
```

225 230 235

<210> 26
<211> 711
<212> DNA
<213> Artificial Sequence

<220>
<223> SF0757 S184Q mutant

<400> 26
atgaaataca aatttatatt attcgacgta gacgatacat tattagattt ccctgaaacg 60

gaaagacacg cattacataa tgcgtttgta cagttcggga tgcctacagg gtataatgat 120

tatcttgcaa gttataaaga gattagtaat ggattatgga gagatttaga aaataaaatg 180

attacgctaa gtgaattagc ggtagatcga tttagacaat tatttgccct tcataatata 240

aaagtagatg cgcagcaatt tagcgatgta tatcttgaaa acttagggaa agaagtacat 300

cttatagaag gtgcagtgca attatgtgag gatctacaag attgcaagtt aggtattatt 360

acgaatggat atacgaaggt gcaacaatcg agaattggaa attcgcctgt atgtaatttc 420

tttgatcata ttattatttc agaagaggtt ggtcatcaaa aaccagcacg tgagattttt 480

gattatgcgt ttgaaaagtt tgggattaca gataaatcaa gtgtattaat ggttggagat 540

tcgctttctc aggatatgag aggcggagaa gattacggca ttgatacgtg ttggtataat 600

ccgagtttga aagaaaatag gacagatgtt aagccgtctt atgaagtgga gagtctgcta 660

caaattttag aaattgtaga agtgactaaa gaaaaagtag cttcatttta a 711

<210> 27
<211> 236
<212> PRT
<213> Artificial Sequence

<220>
<223> SF0757 S184D mutant

<400> 27
Met Lys Tyr Lys Phe Ile Leu Phe Asp Val Asp Asp Thr Leu Leu Asp
1 5 10 15

Phe Pro Glu Thr Glu Arg His Ala Leu His Asn Ala Phe Val Gln Phe
20 25 30

Gly Met Pro Thr Gly Tyr Asn Asp Tyr Leu Ala Ser Tyr Lys Glu Ile
35 40 45

Ser Asn Gly Leu Trp Arg Asp Leu Glu Asn Lys Met Ile Thr Leu Ser
50 55 60

Glu Leu Ala Val Asp Arg Phe Arg Gln Leu Phe Ala Leu His Asn Ile
65 70 75 80

```
Lys Val Asp Ala Gln Gln Phe Ser Asp Val Tyr Leu Glu Asn Leu Gly
                85                  90                  95

Lys Glu Val His Leu Ile Glu Gly Ala Val Gln Leu Cys Glu Asp Leu
            100                 105                 110

Gln Asp Cys Lys Leu Gly Ile Ile Thr Asn Gly Tyr Thr Lys Val Gln
        115                 120                 125

Gln Ser Arg Ile Gly Asn Ser Pro Val Cys Asn Phe Phe Asp His Ile
    130                 135                 140

Ile Ile Ser Glu Glu Val Gly His Gln Lys Pro Ala Arg Glu Ile Phe
145                 150                 155                 160

Asp Tyr Ala Phe Glu Lys Phe Gly Ile Thr Asp Lys Ser Ser Val Leu
                165                 170                 175

Met Val Gly Asp Ser Leu Ser Asp Asp Met Arg Gly Gly Glu Asp Tyr
            180                 185                 190

Gly Ile Asp Thr Cys Trp Tyr Asn Pro Ser Leu Lys Glu Asn Arg Thr
        195                 200                 205

Asp Val Lys Pro Ser Tyr Glu Val Glu Ser Leu Leu Gln Ile Leu Glu
    210                 215                 220

Ile Val Glu Val Thr Lys Glu Lys Val Ala Ser Phe
225                 230                 235


<210>  28
<211>  711
<212>  DNA
<213>  Artificial Sequence

<220>
<223>  SF0757 S184D mutant


<400>  28
atgaaataca aatttatatt attcgacgta gacgatacat tattagattt ccctgaaacg      60

gaaagacacg cattacataa tgcgtttgta cagttcggga tgcctacagg gtataatgat     120

tatcttgcaa gttataaaga gattagtaat ggattatgga gagatttaga aaataaaatg     180

attacgctaa gtgaattagc ggtagatcga tttagacaat tatttgccct tcataatata     240

aaagtagatg cgcagcaatt tagcgatgta tatcttgaaa acttagggaa agaagtacat     300

cttatagaag gtgcagtgca attatgtgag gatctacaag attgcaagtt aggtattatt     360

acgaatggat atacgaaggt gcaacaatcg agaattggaa attcgcctgt atgtaatttc     420

tttgatcata ttattatttc agaagaggtt ggtcatcaaa aaccagcacg tgagattttt     480

gattatgcgt ttgaaaagtt tgggattaca gataaatcaa gtgtattaat ggttggagat     540

tcgctttctg atgatatgag aggcggagaa gattacggca ttgatacgtg ttggtataat     600

ccgagtttga aagaaaatag gacagatgtt aagccgtctt atgaagtgga gagtctgcta     660
```

caaattttag aaattgtaga agtgactaaa gaaaaagtag cttcatttta a 711

<210> 29
<211> 236
<212> PRT
<213> Bacillus cereus

<400> 29

```
Met Lys Tyr Lys Val Ile Leu Phe Asp Val Asp Asp Thr Leu Leu Asp
1               5                   10                  15

Phe Pro Lys Thr Glu Arg His Ala Leu His Asn Ala Phe Val Gln Phe
            20                  25                  30

Asp Met Pro Thr Gly Tyr Asn Asp Tyr Leu Ala Ser Tyr Lys Glu Ile
        35                  40                  45

Ser Asn Gly Leu Trp Arg Asp Leu Glu Asn Lys Met Ile Thr Leu Ser
    50                  55                  60

Glu Leu Ala Val Asp Arg Phe Arg Gln Leu Phe Ala Leu His Asn Ile
65                  70                  75                  80

Asp Val Asp Ala Gln Gln Phe Ser Asp Val Tyr Leu Glu Asn Leu Gly
                85                  90                  95

Lys Glu Val His Leu Ile Glu Gly Ala Val Gln Leu Cys Glu Asn Leu
            100                 105                 110

Gln Asp Cys Lys Leu Gly Ile Ile Thr Asn Gly Tyr Thr Lys Val Gln
        115                 120                 125

Gln Ser Arg Ile Gly Asn Ser Pro Leu Ser Asn Phe Phe Asp His Ile
    130                 135                 140

Ile Ile Ser Glu Glu Val Gly His Gln Lys Pro Ala Arg Glu Ile Phe
145                 150                 155                 160

Asp Tyr Ala Phe Glu Lys Phe Gly Ile Thr Asp Lys Ser Ser Val Leu
                165                 170                 175

Met Val Gly Asp Ser Leu Thr Ser Asp Met Lys Gly Gly Glu Asp Tyr
            180                 185                 190

Gly Ile Asp Thr Cys Trp Tyr Asn Pro Ser Leu Lys Glu Asn Gly Thr
        195                 200                 205

Asp Val Asn Pro Thr Tyr Glu Val Glu Ser Leu Leu Gln Ile Leu Glu
    210                 215                 220

Ile Val Glu Val Ala Glu Glu Lys Val Ala Ser Phe
225                 230                 235
```

<210> 30
<211> 236
<212> PRT
<213> Bacillus cereus

<400> 30

```
Met Lys Tyr Lys Val Ile Leu Phe Asp Val Asp Asp Thr Leu Leu Asp
1               5                   10                  15
```

Phe Pro Lys Thr Glu Arg His Ala Leu His Asn Ala Phe Val Gln Phe
20 25 30

Asp Met Pro Thr Gly Tyr Asn Asp Tyr Leu Ala Ser Tyr Lys Glu Ile
35 40 45

Ser Asn Gly Leu Trp Arg Asp Leu Glu Asn Lys Met Ile Thr Leu Ser
50 55 60

Glu Leu Ala Val Asp Arg Phe Arg Gln Leu Phe Ala Leu His Asn Ile
65 70 75 80

Asp Val Asp Ala Gln Gln Phe Ser Asp Val Tyr Leu Glu Asn Leu Gly
85 90 95

Lys Glu Val His Leu Ile Glu Gly Ala Val Gln Leu Cys Glu Asn Leu
100 105 110

Gln Asp Cys Lys Leu Gly Ile Ile Thr Asn Gly Tyr Thr Lys Val Gln
115 120 125

Gln Ser Arg Ile Gly Asn Ser Pro Leu Ser Asn Phe Phe Asp His Ile
130 135 140

Ile Ile Ser Glu Glu Val Gly His Gln Lys Pro Ala Arg Glu Ile Phe
145 150 155 160

Asp Tyr Ala Phe Glu Lys Phe Gly Ile Thr Asp Lys Ser Ser Val Leu
165 170 175

Met Val Gly Asp Ser Leu Thr Ser Asp Met Lys Gly Gly Glu Asp Tyr
180 185 190

Gly Ile Asp Thr Cys Trp Tyr Asn Pro Ser Leu Lys Glu Asn Gly Thr
195 200 205

Asp Val Asn Pro Thr Tyr Glu Val Glu Ser Leu Leu Gln Ile Leu Glu
210 215 220

Ile Val Glu Val Ala Glu Glu Lys Val Ala Ser Phe
225 230 235

<210> 31
<211> 236
<212> PRT
<213> Bacillus cereus

<400> 31
Met Lys Tyr Lys Val Ile Leu Phe Asp Val Asp Asp Thr Leu Leu Asp
1 5 10 15

Phe Pro Glu Thr Glu Arg His Ala Leu His Asn Ala Phe Val Gln Phe
20 25 30

Asp Met Pro Thr Gly Tyr Asn Asp Tyr Leu Ala Ser Tyr Lys Glu Ile
35 40 45

Ser Asn Gly Leu Trp Arg Asp Leu Glu Asn Lys Met Ile Thr Leu Ser
50 55 60

Glu Leu Ala Val Asn Arg Phe Arg Gln Leu Phe Ala Leu His Asn Ile

```
 65                  70                  75                  80

Asp Val Asp Ala Gln Gln Phe Ser Asp Val Tyr Leu Glu Asn Leu Gly
                85                  90                  95

Lys Glu Val His Leu Ile Glu Gly Ala Val Gln Leu Cys Glu Asn Leu
            100                 105                 110

Gln Asp Cys Lys Leu Gly Ile Ile Thr Asn Gly Tyr Thr Lys Val Gln
        115                 120                 125

Gln Ser Arg Ile Gly Asn Ser Pro Leu Cys Asn Phe Phe Asp His Ile
    130                 135                 140

Ile Ile Ser Glu Glu Val Gly His Gln Lys Pro Ala Arg Glu Ile Phe
145                 150                 155                 160

Asp Tyr Ala Phe Glu Lys Phe Gly Ile Thr Asp Lys Ser Ser Val Leu
                165                 170                 175

Met Val Gly Asp Ser Leu Thr Ser Asp Met Lys Gly Gly Glu Asp Tyr
            180                 185                 190

Gly Ile Asp Thr Cys Trp Tyr Asn Pro Ser Leu Lys Glu Asn Gly Thr
        195                 200                 205

Asp Val Asn Pro Thr Tyr Glu Val Glu Ser Leu Leu Gln Ile Leu Glu
    210                 215                 220

Ile Val Glu Val Ala Glu Glu Lys Val Ala Ser Phe
225                 230                 235


<210>   32
<211>   236
<212>   PRT
<213>   Bacillus cereus

<400>   32
Met Lys Tyr Lys Val Ile Leu Phe Asp Val Asp Asp Thr Leu Leu Asp
  1               5                  10                  15

Phe Pro Glu Thr Glu Arg His Ala Leu His Asn Ala Phe Val Gln Phe
             20                  25                  30

Gly Met Ser Thr Gly Tyr Asn Asp Tyr Leu Ala Ser Tyr Lys Glu Ile
        35                  40                  45

Ser Asn Gly Leu Trp Arg Asp Leu Glu Asn Lys Met Ile Thr Leu Ser
    50                  55                  60

Glu Leu Ala Val Asn Arg Phe Arg Gln Leu Phe Ala Leu His Asn Ile
 65                  70                  75                  80

Asp Val Asp Ala Gln Gln Phe Ser Asp Val Tyr Leu Glu Asn Leu Gly
                85                  90                  95

Lys Glu Val His Leu Ile Glu Gly Ala Val Gln Leu Cys Glu Asn Leu
            100                 105                 110

Gln Asp Cys Lys Leu Gly Ile Ile Thr Asn Gly Tyr Thr Met Val Gln
        115                 120                 125
```

```
Gln Ser Arg Ile Gly Asn Ser Pro Leu Cys Asn Phe Phe Asp His Ile
    130                 135                 140

Ile Ile Ser Glu Glu Val Gly His Gln Lys Pro Ala Arg Glu Ile Phe
145                 150                 155                 160

Asp Tyr Ala Phe Glu Lys Phe Gly Ile Thr Asp Lys Ser Ser Val Leu
                165                 170                 175

Met Val Gly Asp Ser Leu Thr Ser Asp Met Lys Gly Gly Glu Asp Tyr
            180                 185                 190

Ser Ile Asp Thr Cys Trp Tyr Asn Pro Ser Leu Lys Glu Asn Gly Thr
        195                 200                 205

Asp Val Asn Pro Thr Tyr Glu Val Glu Ser Leu Leu Gln Ile Leu Glu
    210                 215                 220

Ile Val Glu Val Ala Glu Glu Lys Val Ala Ser Phe
225                 230                 235


<210>   33
<211>   236
<212>   PRT
<213>   Bacillus cereus

<400>   33
Met Lys Tyr Lys Val Ile Leu Phe Asp Val Asp Asp Thr Leu Leu Asp
1               5                   10                  15

Phe Pro Glu Thr Glu Arg His Ala Leu His Asn Ala Phe Val Gln Phe
            20                  25                  30

Gly Met Pro Thr Gly Tyr Asn Asp Tyr Leu Ala Ser Tyr Lys Glu Ile
        35                  40                  45

Ser Asn Gly Leu Trp Arg Asp Leu Glu Asn Lys Met Ile Thr Leu Ser
    50                  55                  60

Glu Leu Ala Val Asp Arg Phe Arg Gln Leu Phe Ala Leu His Asn Ile
65                  70                  75                  80

Asn Val Asp Ala Gln Gln Phe Ser Asp Val Tyr Leu Glu Asn Leu Gly
                85                  90                  95

Lys Glu Val His Leu Ile Glu Gly Ala Val Gln Leu Cys Glu Asn Leu
            100                 105                 110

Gln Asp Cys Lys Leu Gly Ile Ile Thr Asn Gly Tyr Thr Lys Val Gln
        115                 120                 125

Gln Ser Arg Ile Gly Asn Ser Pro Leu Cys Asn Phe Phe Asp His Ile
    130                 135                 140

Ile Ile Ser Glu Glu Val Gly His Gln Lys Pro Ala Arg Glu Ile Phe
145                 150                 155                 160

Asp Tyr Ala Phe Glu Lys Phe Gly Ile Thr Asp Lys Ser Ser Val Leu
                165                 170                 175

Met Val Gly Asp Ser Leu Thr Ser Asp Met Lys Gly Gly Glu Asp Phe
            180                 185                 190
```

```
Gly Ile Asp Thr Cys Trp Tyr Asn Pro Ser Leu Lys Glu Asn Lys Thr
        195                 200                 205

Ser Val Asn Pro Thr Tyr Glu Val Glu Ser Leu Leu Gln Ile Leu Glu
    210                 215                 220

Ile Val Glu Val Ala Glu Glu Lys Val Ala Ser Phe
225                 230                 235
```

## Description

### BACKGROUND

#### 1. Field

The present disclosure relates to a recombinant microorganism, which includes a foreign gene encoding a variant of a haloacid dehalogenase superfamily protein, a composition including the variant for use in removing a fluorine-compound in a sample, and a method of reducing a concentration of a fluorine-containing compound in a sample using the haloacid dehalogenase superfamily protein.

#### 2. Description of the Related Art

The emission of greenhouse gases, which have accelerated global warming, is a serious environmental problem, and regulations to reduce and prevent the emissions of greenhouse gases have been tightened. Among the greenhouse gases, fluorinated gases (F-gases), such as perfluorocarbons (PFCs), hydrofluorocarbons (HFCs), and sulfur hexafluoride ($SF_6$), show low absolute emission, but have a long half-life and a very high global warming potential, resulting in significantly adverse environmental impact. The amount of F-gases emitted from the semiconductor and electronics industries, which are major causes of F-gas emission, has exceeded the assigned amount of greenhouse gas emissions and continues to increase. Therefore, costs required for decomposition of greenhouse gases and greenhouse gas emission allowances are increasing every year.

A pyrolysis or catalytic thermal oxidation process has generally been used in the decomposition of F-gases. However, this process has disadvantages of limited decomposition rate, emission of secondary pollutants, and high cost. However, biological decomposition of F-gases would allow F-gases to be treated in a more economical and environmentally-friendly manner.

Therefore, there is a need to develop new microorganisms and methods for the biological decomposition of F-gases. This invention provides such microorganisms and methods.

### SUMMARY

Provided is a variant haloacid dehalogenase superfamily protein, as well as a polynucleotide encoding the variant.

Also provided herein is a recombinant microorganism including a foreign gene encoding the variant haloacid dehalogenase superfamily protein.

Further provided is a composition for use in reducing a fluorine-containing compound in a sample, the composition including the variant haloacid dehalogenase superfamily protein or recombinant microorganism expressing same.

Also provided is a method of reducing a concentration of a fluorine-containing compound in a sample, the method including contacting the variant of a haloacid dehalogenase superfamily protein with a sample including a fluorine-containing compound, so as to reduce the concentration of the fluorine-containing compound in the sample, wherein the variant haloacid dehalogenase protein is, optionally, comprised in a recombinant microorganism that expresses the protein.

### BRIEF DESCRIPTION OF THE DRAWINGS

These and/or other aspects will become apparent and more readily appreciated from the following description of the embodiments, taken in conjunction with the accompanying drawings in which:

FIG. 1 is a vector map of a pET-BC3334 vector;
FIG. 2 is a schematic diagram of a glass Dimroth reflux condenser;
FIG. 3 is a vector map of a pET-SF0757 vector; and
FIG. 4 shows alignment results of homologous sequence of a BC3334 protein.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects. As used herein, the term "and/or" includes any and all combinations of one or more of the associated listed items.

The term "gene" as used herein refers to a polynucleotide that expresses a particular protein. A gene may include regulatory sequences, such as a coding region sequence and a non-coding region including a 5' non-coding sequence and a 3' non-coding sequence. The regulatory sequence may also include a promoter, an enhancer, an operator, a ribosome binding site, a polyA binding site, a terminator region, and the like.

The term "sequence identity" as used herein with respect to a nucleic acid or a polypeptide refers to a degree of identity between bases or amino acid residues of sequences after being aligned to best match in a certain comparative region. The sequence identity is a value measured by comparing two sequences in a certain comparative region through optimal alignment of the two sequences, wherein some portions of the sequences in the comparative region may be added or deleted compared to a reference sequence. A percentage of sequence identity may be for example, calculated as follows: two sequences that are optimally aligned are compared in the entire comparative region; the number of locations where the same amino acids or nucleic acids appear in both sequences is determined to the number of matching locations; the number of matching locations is divided by the total number of locations (i.e., the size of a range) in the comparative region; and the result of the division is multiplied by 100 to obtain the percentage of the sequence identity. The percentage of the sequence identity may be determined using a known sequence comparison program, such as BLASTN or BLASTP (NCBI), CLC Main Workbench (CLC bio), or MegAlign™ (DNASTAR Inc). Unless otherwise mentioned in the specification, the selection of the parameters used to execute the program may be as follows: E-value=0.00001 and H-value=0.001.

An aspect of the present invention provides a recombinant microorganism including a foreign gene encoding a variant of a haloacid dehalogenase (HAD) superfamily protein.

A HAD superfamily protein may be an enzyme including phosphatase, phosphonatase, P-type ATPase, beta-phosphoglucomutase, phosphomannomutase, and dehalogenase. The HAD superfamily protein may include a HAD domain. The HAD superfamily protein may be phospholipid-translocating ATPase belonging to EC 3.6.3.1, 3-deoxy-D-manno-octulosonate (KDO) 8-phosphate phosphatase belonging to EC 3.1.3.45, mannosyl-3-phosphoglycerate phosphatase belonging to EC 3.1.3.70, phosphoglycolate phosphatase belonging to EC 3.1.3.18, or HAD belonging to EC 3.8.1.2.

The ATPase may be a putative lipid-flipping enzyme involved in cold tolerance in *Arabidopsis.* The 3-deoxy-D-manno-octulosonate (KDO) 8-phosphate phosphatase may catalyze the final step in the biosynthesis of KDO, which is a component of lipopolysaccharide in Gram-negative bacteria. The mannosyl-3-phosphoglycerate phosphatase may hydrolyse mannosyl-3-phosphoglycerate to form osmolyte mannosylglycerate. The phosphoglycolate phosphatase may catalyze the dephosphorylation of 2-phosphoglycolate.

The HAD superfamily protein thereof may have a sequence identity of 85% or more, 90% or more, 95% or more, 96% or more, 97% or more, 98% or more, or 99% or more with respect to an amino acid sequence of SEQ ID NO: 1, 5, 29, 30, 31, 32, or 33.

The variant may be a HAD superfamily protein as described above with an amino acid alteration in at least one amino acid residue corresponding to positions N122 and S184, of SEQ ID NO: 1. Thus, for instance, the variant HAD superfamily protein may have a sequence identity of 85% or more, 90% or more, 95% or more, 96% or more, 97% or more, 98% or more, or 99% or more with respect to an amino acid sequence of SEQ ID NO: 1, 5, 29, 30, 31, 32, or 33 provided the sequence comprises an alteration in one or more amino acid residues corresponding to N122 or S184. The variant itself may be an enzyme belonging to the HAD superfamily, for example, an enzyme having the activity of a dehalogenase belonging to EC 3.8.1.2. The amino acid alteration may include a substitution of V or Y for N122, a substitution of H, Q, or D for S184, or a combination thereof. Alternatively, the alteration may be a conservative substitution for V or Y at position 122, or a conservative substitution for H, Q, or D at position 184. In other words, the alteration may be a substitution of the amino acid corresponding to N122 with an amino acid that is conservative with respect to V or Y, or a substitution of the amino acid corresponding to S184 with an amino acid that is conservative with respect to H, Q, or D. A conservative substitution for H at position 184 may be S184K, or S184R. A conservative substitution for Q at position 184 may be S184T, S184C, S184Y,or S184N. A conservative substitution for D at position 184 may be S184E. A conservative substitution for V at position 122 may be N122G, N122A, N122L, N122I, N122M, N122F, N122W, or N122P. A conservative substitution for Y at position 122 may be N122S, N122T, N122C, or N122Q.

The variant may be prepared by substituting at least one residue, which corresponds to N122 and S184 in the HAD superfamily protein BC3334 having the amino acid of SEQ ID NO: 1, with another amino acid, for example, one of the 19 natural amino acids. The variant may be prepared by substitution of V or Y for an amino acid residue corresponding to residue N122 position in BC3334 having amino acid sequence of SEQ ID NO: 1. The variant may be prepared by substitution of H, Q, or D for an amino acid residue corresponding to residue S184 of the amino acid sequence of SEQ ID NO: 1. The amino acid residues corresponding to the N122 position and the S184 position in BC3334 of the amino acid sequence of SEQ ID NO: 1 may each be an amino acid residue corresponding to the N122 position and an amino acid residue corresponding to the S184 position in SF0757 of an amino acid sequence of SEQ ID NO: 5.

The variant may have the substitution N122V or N122Y in the amino acid sequence of SEQ ID NO: 1, or in some embodiments, may have S184H, S184Q, or S184D, in the amino acid sequence of SEQ ID NO: 5. In other words, the variant can comprise SEQ ID NO: 1 in which N122 is substituted with V or Y, or SEQ ID NO: 5 in which S184 is substituted with H, Q, or D.

The variant may be a single variant, such as a N122V, N122Y, S184H, S184Q, or S184D (e.g., of any of SEQ ID NOs: 1, 5, 29, 30, 31, 32, or 33), or a double variant, such as N122V and S184H; N122V and S184Q; N122V and S184D; N122Y and S184H; N122Y and S184Q; or N122Y and S184D (e.g., of any of SEQ ID NOs: 1, 5, 29, 30, 31, 32, or 33). In some embodiments, the variant HAD protein comprises SEQ ID NO: 19, 21, 23, 25, or 27, or a sequence with 85%, 90%, 95%, or 99% sequence identity thereto.

The amino acid alteration may include substitution, insertion, or deletion. The substitution may include substitution with an amino acid that is modified after translation. The substitution may include substitution with one of 19 amino acids other than the corresponding amino acid among 20 natural amino acids. Amino acids used herein and abbreviations thereof are shown in Table 1.

[Table 1]

| Abbreviation | | Amino acid |
|---|---|---|
| A | Ala | Alanine |
| C | Cys | Cysteine |
| D | Asp | Aspartic acid |
| E | Glu | Glutamic acid |
| F | Phe | Phenylalamine |
| G | Gly | Glycine |
| H | His | Histidine |
| I | Ile | Isoleucine |
| K | Lys | Lysine |
| L | Leu | Leucine |
| M | Met | Methionine |
| N | Asn | Asparagine |
| P | Pro | Proline |
| Q | Gln | Glutamine |
| R | Arg | Arginine |
| S | Ser | Serine |
| T | Thr | Threonine |
| V | Val | Valine |
| W | Trp | Tryptophan |
| Y | Tyr | Tyrosine |

The term "conservative" or "conservative substitution" as used herein refers to substitution of an amino acid with a similar amino acid in terms of the amino acid characteristics. For example, when a non-aliphatic amino acid residue (e.g., Ser) at a specific position is substituted with an aliphatic amino acid residue (e.g., Leu), a substitution with a different aliphatic amino acid (e.g., lie or Val) at the same position is referred to as a conservative mutation. In addition, the amino acid characteristics include size of the residue, hydrophobicity, polarity, charge, pK-value, and other amino acid characteristics known in the art. Accordingly, a conservative mutation may include substitution, such as basic for basic, acid for acid, polar for polar, and the like. Conservative substitutions may be made, for example, according to Table 2 below which describes a generally accepted grouping of amino acid characteristics.

[Table 2]

| Set | Amino acids |
|---|---|
| Non-polar | G A V L I M F W P |
| Polar | S T C Y N Q |
| Acidic | D E |
| Basic | K R H |

The term "corresponding" as used herein refers to the amino acid position of a protein of interest that aligns with the mentioned position of a reference protein (e.g., position N122 or S184 of SEQ ID NO: 1) when amino acid sequences of the protein of interest and the reference protein are aligned using an art-acceptable protein alignment program, such as the BLAST pairwise alignment or the well known Lipman-Pearson Protein Alignment program. For example, the amino acid residues at the positions N122 and S184 of the amino acid sequence of SEQ ID NO: 1 may each correspond to the amino acid residue of a protein of interest, for example, position N122 and position S184 of the amino acid sequence of SEQ ID NO: 5. The protein of interest may be HAD, which belongs to, for example, EC 3.8.1.2. The database (DB) in which the reference sequence is stored may be the Reference Sequence (RefSeq) non-redundant protein database of NCBI. The parameters used for the sequence alignment may be as follows: E-value 0.00001 and H-value 0.001.

Examples of the proteins obtained according to the alignment conditions above and having the amino acid residues corresponding to positions N122 and S184 of the amino acid sequence of SEQ ID NO: 1 (hereinafter, referred to as "homologs of BC3334") are shown in Table 3 below. The homologs may have 85% or more sequence identity to the amino acid sequence of SEQ ID NO: 1. The results of aligning the sequences and the numbering of the sequences are shown in FIG. 4. In FIG. 4, the underlined parts represent the positions N122 and S184, the N-terminal residue is 1, and the C-terminal residue is 236.

[Table 3]

| SEQ ID NO: | Gene symbol (Locus tag) | Gene description |
|---|---|---|
| 29 | CT43_CH3258 | 2-haloalkanoic acid dehalogenase<br>Bacillus thuringiensis serovar chinensis CT-43 |
| 30 | BTB_c33930 | yfnB1: tentative HAD-hydrolase YfnB<br>Bacillus thuringiensis Bt407 |
| 31 | BMB171_C3020 | 2-haloalkanoic acid dehalogenase<br>Bacillus thuringiensis BMB171 |
| 32 | BCB4264_A3346 | Hydrolase, HAD-like family<br>Bacillus cereus B4264 |
| 33 | BTG_02795 | 2-haloalkanoic acid dehalogenase<br>Bacillus thuringiensis HD-771 |

The recombinant microorganism may be bacteria or fungi, and the bacteria may be Gram-positive or Gram-negative. The Gram-negative bacteria may belong to the Enterobacteriaceae family. The Gram-negative bacteria may belong to the genus *Escherichia,* the genus *Samonella,* the genus *Xanthobacter,* or the genus *Pseudomonas.* The microorganism belonging to the genus *Escherichia* may be *E. coli.* The microorganism belonging to the genus *Xanthobacter* may be *X. autotrophicus.* The Gram-positive bacteria may belong to the genus *Corynebacterium* or the genus *Bacillus.* The recombinant microorganism may include at least one foreign or heterologous polynucleotide encoding a variant HAD superfamily protein as described herein, for example, a polynucleotide encoding SEQ ID NO: 19, 21, 23, 25, or 27, or having a nucleotide sequence of SEQ ID NO: 20, 22, 24, 26, or 28.

Another aspect of the invention provides a composition including a variant of a haloacid dehalogenase (HAD) superfamily protein, as described herein, for use in removing a fluorine-containing compound from a sample. In certain embodiments the composition may comprise a fluorine-containing compound, such as those described herein.

The fluorine-containing compound may be represented by Formula 1 or 2:

<Formula 1> $C(R^1)(R^2)(R^3)(R^4)$

<Formula 2> $(R^5)(R^6)(R^7)C-[C(R^{11})(R^{12})]n-C(R^8)(R^9)(R^{10})$.

In Formulae 1 and 2, n may be an integer from 0 to 10; $R^1$, $R^2$, $R^3$, and $R^4$ may each independently be fluorine (F), chlorine (Cl), bromine (Br), iodine (I), or hydrogen (H), provided at least one of $R^1$, $R^2$, $R^3$, or $R^4$ is F; $R^5$, $R^6$, $R^7$, $R^8$, $R^9$, $R^{10}$, $R^{11}$, and $R^{12}$ may each be independently F, Cl, Br, I, or H, provided that at least one of $R^5$, $R^6$, $R^7$, $R^8$, $R^9$, $R^{10}$, $R^{11}$, or $R^{12}$ is F.

The fluorine-containing compound may be, for example, $CHF_3$, $CH_2F_2$, $CH_3F$, or $CF_4$. The term "removal" as used herein refers to reduction of the concentration of the fluorine-containing compound in the sample, and the reduction includes partial or complete removal.

The variant of the HAD protein may be provided in the composition by a recombinant microorganism that expresses the variant, i.e., a recombinant microorganism that includes a foreign gene that encodes the variant of the HAD protein. The composition may include the recombinant microorganism itself, a lysate thereof, or an aqueous material fraction of the lysate. The recombinant microorganism for use in the composition may be any recombinant microorganism described herein.

The removal of the fluorine-containing compound from the sample encompasses any reduction of the concentration of the fluorine-containing compound in the sample, which may be achieved by cleavage of a C-F bond of the fluorine-containing compound, conversion of the fluorine-containing compound into a different material, or accumulation of the fluorine-containing compound in a cell. The conversion of the fluorine-containing compound may include introduction of a hydrophilic group, such as a hydroxyl group, to the fluorine-containing compound, or introduction of a carbon-carbon double bond or a carbon-carbon triple bond to the fluorine-containing compound.

The sample may be a liquid sample or a gaseous sample. The sample may be, for instance, industrial sewage or waste gas.

Another aspect of the invention provides a method of reducing a concentration of a fluorine-containing compound in a sample, the method including contacting the variant haloacid dehalogenase (HAD) superfamily protein described herein (or composition comprising same or microorganism expressing same) with a sample including a fluorine-containing compound, so as to reduce the concentration of the fluorine-containing compound in the sample. The term "removal" as used herein refers to reduction of the concentration of the fluorine-containing compound in the sample, and includes partial or complete removal.

Contacting of the variant HAD superfamily protein with the fluorine-containing sample may be performed in any manner. In one embodiment, the contacting is performed in an air-tight closed container. The contacting may be gas-liquid contacting of a gaseous sample with a liquid containing the variant of the HAD protein. In addition, the contacting may be liquid-liquid contacting of a liquid sample with a liquid containing the variant of the HAD protein. The contacting may include mixing.

The variant of the HAD protein for use in the inventive method may be included in a recombinant microorganism including a foreign gene that encodes the variant of the HAD protein. In this regard, the contacting may include contacting the sample with a cell first, and then, with the variant protein in the cell. The variant protein may be provided in the recombinant microorganism, a lysate thereof, or an aqueous material fraction of the lysate. Contacting the recombinant microorganism comprising the HAD superfamily protein with the sample comprising a fluorine-containing compound may be performed under conditions where the recombinant microorganism may survive in an air-tight closed container. Such conditions for the survival of the recombinant microorganism may include conditions where the recombinant microorganism may proliferate or conditions where the recombinant microorganism may be allowed to be in a resting state. In this regard, the contacting may include culturing a microorganism in the presence of the fluorine-containing compound. The culturing may be performed under aerobic or anaerobic conditions.

The sample of the inventive method may be a liquid sample or a gaseous sample. The sample may be industrial sewage or waste gas.

Another aspect of the invention provides a variant haloacid dehalogenase (HAD) superfamily protein itself. The variant protein can be any of the variant HAD superfamily proteins described herein with respect to the other aspects of the disclosure. Generally, the variant HAD protein includes an amino acid alteration in at least one amino acid residue corresponding to positions N122 and S184 of SEQ ID NO: 1.

Another aspect of the invention provides a polynucleotide encoding a variant haloacid dehalogenase (HAD) superfamily protein. The polynucleotide and the variant HAD protein encoded thereby can be any previously described herein with respect to the other aspects of the invention. The polynucleotide encoding the variant may be included in a vector. For use as a vector, any vehicle that can be used to introduce the polynucleotide to a microorganism may be used. The vector may be a plasmid vector or a viral vector. The vector may be in a recombinant microorganism as described herein.

Hereinafter, the present invention will be described in more detail with reference to Examples. However, these Examples are provided for illustrative purposes only, and the invention is not intended to be limited by these Examples.

**Example 1: Recombinant *E.* coli expressing BC3334 gene and removal of a fluorine-containing compound in a sample using the recombinant *E.* coli**

Recombinant *E. coli* expressing a HAD geneor a gene of a variant of the HAD gene was prepared, and the effect on the removal of $CF_4$ in a sample was confirmed.

**1. Amplification of a HAD gene (BC3334) from *B. cereus* and introduction of the gene into *E. coli***

The BC3334 gene from *B. cereus* (ATCC 14579) was amplified. For amplification of the BC3334 gene, PCR was performed using the genomic DNA of the strain as a template and a set of primers having the nucleotide sequences of SEQ ID NOs: 3 and 4. The amplified BC3334 gene was ligated with a pETDuet-1 (Novagen, Cat. No. 71146-3), which was digested with restriction enzymes, NcoI and HindIII, using the InFusion Cloning Kit (Clontech Laboratories, Inc.), thereby preparing a pET-BC3334 vector. FIG. 1 is a vector map of the pET-BC3334. The BC3334 protein has an amino acid sequence of SEQ ID NO: 1, and is encoded by the nucleotide sequence of SEQ ID NO: 2.

Next, the pET-BC3334 vector was introduced into *E. coli* BL21 by a heat shock method, and then, cultured in an LB plate containing 100 μg/mL of ampicillin. Strains showing ampicillin resistance were selected. Then, a selected strain was designated as a recombinant *E. coli* BL21/pET-BC3334wt.

**2. Recombinant *E. coli* expressing a variant of the BC3334**

A variant of the HAD protein BC3334 was prepared to improve the activity of the BC3334 protein on the removal of a fluorine-containing compound in a sample. Asparagine at position 122 (hereinafter, referred to as "N122") of the amino acid sequence of SEQ ID NO: 1 and/or serine at position 184 (hereinafter, referred to as "S184") was substituted with one of the other 19 natural amino acids. Here, each substitution is represented by "N122X" (wherein X indicates each of 19 natural amino acids other than asparagines) or "S184X" (wherein X indicates each of 19 natural amino acids other than serine). The effect of *E. coli,* which was prepared by introducing a gene encoding the prepared variant thereto, on the removal of $CF_4$ in a sample was confirmed.

The preparation of the N122X and/or S184X variant of SEQ ID NO: 1 was achieved by using the QuikChange II Site-Directed Mutagenesis Kit (Agilent Technology, USA). Site-directed mutagenesis using the kit was performed by using *PfuUlta* high-fidelity (HF) DNA polymerase for mutagenic primer-directed replication of two plasmid strands with the highest fidelity. The basic procedure utilizes a super-coiled double-stranded DNA (dsDNA) vector with an insert of interest and two synthetic oligonucleotide primers, both containing the desired mutation. The oligonucleotide primers, each complementary to opposite strands of the vector, were extended during temperature cycling by PfuUltra HF DNA polymerase, without primer displacement. Extension of the oligonucleotide primers generated a mutated plasmid containing staggered nicks. Following temperature cycling, the product was treated with *DpnI.* The *DpnI* endonuclease (target sequence: 5'-Gm$^6$ATC-3') was specific for methylated and hemimethylated DNA, and was used to digest the parental DNA template for the selection of mutation-containing synthesized DNA. Afterwards, the nicked vector DNA incorporating the desired mutations was then transformed into XL1-Blue supercompetent cells.

Among the primer sets used to induce mutagenesis of N122X and S184X, primer sets of SEQ ID NOs: 9 and 10 and primer sets of SEQ ID NOs: 11 and 12 were each used for N122Y and N122V. BC3334 proteins having the N122Y variation or having the N122V variation each had an amino acid sequence of SEQ ID NOs: 19 and 21, each of which amino acid sequences was encoded by a nucleotide sequence of SEQ ID NO: 20 and a nucleotide sequence of SEQ ID NO: 22.

In detail, PCR was performed by using the pET-BC3334wt vector prepared in section (1) as a template and the primer sets for each of the variants as a primer, and a *PfuUlta* HF DNA polymerase to obtain variant vectors including staggered nicks. These vector products were treated with *DpnI* to select variant-containing synthesized DNA. Afterwards, the nicked vector DNA incorporating a desired variant was then transformed into XL1-Blue supercompetent cells, thereby cloning the pET-BC3334mt vector.

Lastly, the cloned pET-BC3334mt vector was introduced to a strain of *E. coli* BL21 in the same manner as in section (1), and a finally selected strain was designated as a recombinant *E. coli* BL21/pET-BC3334mt.

**3. Effect of recombinant *E. coli* including a BC3334 variant introduced thereto on the removal of $CF_4$ in a sample**

The effect of the *E. coli* BL21/pET-BC3334mt prepared in section (2) and including the mutant BC3334 gene introduced thereto on the removal of $CF_4$ in a sample was confirmed.

In detail, a strain of the *E. coli* BL21/pET-BC3334mt was cultured in a LB medium with stirring at a temperature of 30°C at a speed of 230 rpm, and at an $OD_{600}$ of about 0.5, IPTG 0.2 mM was added to the medium, followed by being cultured overnight with stirring at a temperature of 20°C at a speed of 230 rpm. Then, the cells were harvested and suspended in a LB medium, so as to have a cell concentration $OD_{600}$ of 3.0. 10 mL of the cell solution was added to a 60 mL serum bottle, and the serum bottle was sealed. The LB medium was supplemented with 10 g of tripton per 1 L of distilled water, 5 g of an enzyme extract, and 10 g of NaCl. Next, $CF_4$ in a gas phase was injected into the serum bottle through a rubber stopper of a cap of the serum bottle with a syringe, so as to have 1,000 ppm of $CF_4$ in a head space of the serum bottle. Afterwards, the serum bottle was cultured for 4 days with stirring at a temperature of 30°C at a speed of 230 rpm. The experiments were performed in triplicate. After incubation, 0.5 mL of $CF_4$ gas was collected by using a 1.0 mL syringe from the head space, which did not contain the medium, of the serum bottle, and then, was injected into a gas chromatograph (GC) column (Agilent 7890, Palo Alto, CA, USA). The injected $CF_4$ gas was separated by a CP-PoraBOND Q column (25 m length, 0.32 mm inner diameter, 5 μm film thickness, Agilent), and changes in the concentration of $CF_4$ gas was analyzed by mass spectrometry (MS) (Agilent 5973, Palo Alto, CA, USA). Here, helium was used as a carrier gas, and was flowed into the column at a rate of 1.5 ml/min. Regarding conditions for the GC, a temperature at an inlet was 250°C, and an initial temperature was maintained at 40°C for 2 minutes and raised up to 290°C at a speed of 20°C/min. Regarding conditions for the MS, an ionization energy was 70 eV, an interface temperature was 280°C, an ion source temperature was 230°C , and a quadrupole temperature was 150°C. As a result, in Table 4, strains including the variant showed the activity of removing $CF_4$ gas in a sample as compared to a wild type.

[Table 4]

| Strain | Decomposition rate of $CF_4$ (%, as compared to a control group) |
|---|---|
| Wild type | 4.11 |
| BL21/pET-BC3334mt(N122Y) | 9.29 |
| BL21/pET-BC3334mt(N122V) | 5.99 |

In Table 4, the control group was *E. coli* BL21 to which an empty pETDuet vector was introduced instead of the pET-BC3334mt vector, and the wild type was *E. coli* including wild type BC3334 gene.

As shown in Table 4, the *E. coli* including the wild type BC3334 gene showed a reduction of $CF_4$ by 4.11 % as compared to the control group, wherein the *E. coli* including the variants of the BC3334 gene, i.e., the N122Y and N122V genes, showed a reduction of $CF_4$ by 9.29% and 5.99%, respectively, as compared to the control group.

**4. Decomposition of a fluorine-containing compound by a circulation process**

As shown in FIG. 2, 50 ml of an LB medium and 1,000 ppm of $CF_4$ gas were added to a glass Dimroth coil reflux condenser (a reactor length: 350 mm, an exterior diameter: 35 mm, and an interior volume: 200 mL) that was sterilized and vertically oriented, and then, the LB medium was subjected to circulation. FIG. 2 is a schematic diagram of the glass Dimroth reflux condenser (10). The LB medium was supplied to an inlet (12) of an upper portion of the condenser (10), flowed through an inner wall of the condenser (10), and discharged to an outlet (14) of a lower portion of the condenser (10). The discharged LB medium was re-supplied to the inlet (12) along a circulation line (18). Although not shown in FIG. 2, to maintain the temperature, an inner screwed pipe of the condenser (10) was connected to a constant temperature zone of 30°C. The circulation is performed by a pump (16). Here, the circulation rate of the LB medium was maintained at 4 mL/min. After an appointed period of time, i.e., 0, 48, 96, and 144 hours, the amount of the $CF_4$ gas in the condenser was confirmed by gas chromatography mass-spectrum (GC-MS). Then, it was confirmed that there was no change in the amount of $CF_4$ gas.

Subsequently, the recombinant microorganisms of sections (1) to (3) and the control group were each inoculated on an LB medium in the condenser (10) by using a syringe, so as to have an initial concentration of 5.0 on the basis of $OD_{600}$. The recombinant microorganism was *E. coli* to which a wild type BC3334 gene was introduced and *E. coli* to which a gene of the BC3334 variant was introduced. Then, the *E.* coli to which the wild type BC3334 gene was introduced and the *E. coli* to which the gene of the BC3334 variant was introduced were subjected to comparison of the $CF_4$ decomposition capability. The *E. coli* to which the empty vector was introduced was used as a negative control group, and there was no change in the level of $CF_4$.

Here, the circulation rate of the LB culture medium was about 4 mL/min, and the temperature inside the condenser (10) was maintained at 30°C. After the strain inoculation and the elapse of 144 hours, the amount of $CF_4$ gas in the condenser (10) was confirmed by GC-MS. Then, the decomposition rate of $CF_4$ was calculated according to Equation 1, and the results are shown in Table 5.

< Equation 1>

Decomposition rate of $CF_4$ = [(Initial amount of $CF_4$ – amount of $CF_4$ after reaction) / initial amount of $CF_4$] × 100

[Table 5]

| Strain | Decomposition rate of $CF_4$ (%) |
|---|---|
| BL21/pET-BC3334wt | 33.1 |
| BL21/pET-BC3334mt(N122Y) | 44.5 |

As shown in Table 5, the BC3334 variant after 144 hours of the culture showed an increase in the degradation rate by 1.34 times the degradation rate of the wild type strain, when applying the gas phase circulation process using the microorganism thereto.

**Example 2: Removal of $CF_4$ in a sample by a recombinant *E. coli* expressing SF0757 gene and a gene of a variant thereof**

**1. Selection of a strain of *Bacillus bombysepticus* SF3 and decomposition of a fluorine-containing compound by the strain**

In the present example, a microorganism capable of reducing a concentration of $CF_4$ in industrial wastewater was selected.

The sludge of the wastewater discharged from the plant of Samsung Electronics (Giheung, Korea) was smeared on an agar plate including a carbon-free agar plate (an agar medium supplemented with 0.7 g/L of $K_2HPO_4$, 0.7 g/L of $MgSO_4 \cdot 7H_2O$, 0.5 g/L of $(NH_4)_2SO_4$, 0.5 g/L of $NaNO_3$, 0.005 g/L of NaCl, 0.002 g/L of $FeSO_4 \cdot 7H_2O$, 0.002 g/L of $ZnSO_4 \cdot 7H_2O$, 0.001 g/L of $MnSO_4$, and 15 g/L of agar), and the agar plate was added to a GasPak™ Jar (BD Medical Technology). The inside of the jar was filled with 99.9 v/v% $CF_4$, and the jar was sealed for the standing culture at a temperature of 30°C under anaerobic conditions. Single colonies formed after the culturing were cultured using a high throughput screening (HTS) system (Thermo Scientific/Liconic/Perkin Elmer). Each cultured single colony was inoculated on a 96-well microplate containing 100 $\mu$L of medium per well, and then, was subjected to static culture at a temperature of about 30°C for 96 hours under aerobic conditions. Meanwhile, the growth ability of the colonies was observed by measuring the absorbance at 600 nm every 12 hours.

The top 2% of strains showing excellent growth ability were selected and were each inoculated in a glass serum bottle (volume of 75 mL) containing 10 mL of the LB medium, so as to have an $OD_{600}$ of 0.5. The glass serum bottle was sealed, and then, $CF_4$ gas was injected thereto by using a syringe, so as to have 1,000 ppm of $CF_4$ gas. The glass serum bottle was incubated in a shaking incubator for 4 days at a temperature of 30°C with stirring at a speed of 230 rpm, and then, the amount of $CF_4$ in a head space was analyzed.

For the analysis, 0.5 ml of the headspace gas in the glass serum bottle was collected using a syringe, and then, the amount of $CF_4$ was analyzed under the same conditions as described in section (3). In the case of the control group, 1,000 of $CF_4$ having no cells was incubated and measured under the same conditions as described above.

Consequently, compared to the control group having no cells, the concentration of $CF_4$ was reduced by 10.27% in the separated microorganisms. The microorganisms had decomposition activity of 0.02586 g/kg-cell/h. To identify the selected strains, the genome sequences thereof were analyzed.

A genome obtained by assembling 3 contigs that were obtained by next generation sequencing (NGS) had a final size of 5.3 Mb, and as a result of gene annotation, a total of 5,490 genes were found to be present. As a result of phylogenetic tree analysis performed on each contig, it was confirmed that the microorganism belonged to *Bacillus bombysepticus.*

The separated microorganism was newly named as *Bacillus bombysepticus* SF3, deposited at the Korean Collection for Type Culture (KCTC), which is an international depository authority under the Budapest Treaty, on February 24, 2017, and assigned the accession number of KCTC 13220BP.

**2. Preparation of a recombinant microorganism including a gene derived from a strain of *B. bombysepticus* SF3 and a variant thereof**

By the genomic sequence analysis of the strain of B. *bombysepticus* SF3 identified as described in section (1), genes presumed to encode dehalogenase, such as SF0757 (SEQ ID NO: 6), was selected.

B. *bombysepticus* SF3 was cultured overnight in an LB medium with stirring at a temperature of 30°C at a speed of 230 rpm, and genomic DNA thereof was isolated using a total DNA extraction kit (Invitrogen Biotechnology). PCR was performed using the genome DNA as a template and a set of primers having nucleotide sequences of SEQ ID NOs: 7 and 8, as so to amplify a F0757 gene. The genes thus amplified were ligated with a pET28a vector (Novagen, Cat. No. 69864-3), respectively which was digested with restriction enzymes, such as NcoI and XhoI, by using an InFusion Cloning Kit (Clontech Laboratories, Inc.), so as to prepare a pET-SF0757 vector. FIG. 3 is a vector map of the pET-SF0757 vector. Here, the SF0757 gene had a nucleotide sequence of SEQ ID NO: 6, and encoded an amino acid sequence of SEQ ID NO: 5.

Next, the prepared pET-SF0757 vector was introduced to *E. coli* BL21 by a heat shock method, and then, cultured in an LB plate agar supplemented with 50 $\mu$g/mL of kanamycin. Strains showing kanamycin resistance were then selected, and a finally selected strain was designated as a recombinant *E. coli* BL21/pET-SF0757.

**3. Preparation of a recombinant *E. coli* expressing a SF0757 variant**

In this section, a variant was prepared to improve the activity of the SF0757 gene on the removal of a fluorine-containing compound in a sample. Asparagine at position 122 (hereinafter, referred to as "N122") of the amino acid sequence of SEQ ID NO: 5 and/or serine at position 184 (hereinafter, referred to as "S184") was substituted with other 19 natural amino acids. Here, each substitution is represented by "N122X" (wherein X indicates each of 19 natural amino acids other than asparagines) or "S184X" (wherein X indicates each of 19 natural amino acids other than serine). The effect of *E. coli,* which was prepared by introducing a gene encoding the prepared variant thereto, on the removal of $CF_4$ in a sample was confirmed.

Among the primer sets used to induce mutagenesis of N122X and S184X, primer sets of SEQ ID NOs: 13 and 14, primer sets of SEQ ID NOs: 15 and 16, and primer sets of SEQ ID NOs: 17 and 18 were used for S184H, S184Q, and S184D. The SF0757 protein having the S184H, S184Q, and S184D variants each had an amino acid sequence of SEQ ID NOs: 23, 25, and 27, each of which amino acid sequences was encoded by a nucleotide sequence of SEQ ID NOs: 24, 26, and 28.

The preparation of the variant and the recombinant *E. coli* including a gene of the variant are the same as described in section (3) of Example 1.

Lastly, the cloned pET-SF0757mt vector was introduced to a strain of *E. coli* BL21 in the same manner as in section (1), and a finally selected strain was designated as a recombinant *E. coli* BL21/pET-SF0757mt.

**4. Effect of recombinant *E. coli* including a SF0757 variant introduced thereto on the removal of $CF_4$ in a sample**

The influence of the *E. coli* BL21/pET-SF0757mt prepared in section (3) to which the SF0757 variant was introduced on the removal of $CF_4$ in a sample.

In detail, a strain of *E. coli* BL21/pET-SF0757mt was cultured in an LB medium with stirring at a temperature of 30°C at a speed of 230 rpm, and at an $OD_{600}$ of about 0.5, IPTG 0.2 mM was added to the medium, followed by being cultured overnight with stirring at a temperature of 20°C at a speed of 230 rpm. Then, the cells were harvested and suspended in a LB medium, so as to have a cell concentration $OD_{600}$ of 3.0. 10 mL of the cell solution was added to a 60 mL serum bottle, and the serum bottle was sealed. Next, $CF_4$ in a gas phase was injected to the serum bottle through a rubber stopper of a cap of the serum bottle by using a syringe, so as to have 1,000 ppm of $CF_4$ in a head space of the serum bottle. Afterwards, the serum bottle was cultured for 4 days with stirring at a temperature of 30°C at a speed of 230 rpm. The experiments were performed in triplicate. After incubation, $CF_4$ gas was collected from the head space, which did not contain the medium, of the serum bottle, and then, analyzed under the same conditions as described in section (3) of Example 1.

Consequently as shown in Table 6, the strains including the variants above exhibited increased activity of removing $CF_4$ gas in the sample as compared to the wild type strain.

[Table 6]

| Strain | Decomposition of $CF_4$ (%, as compared to control group) |
|---|---|
| Wild type | 3.95 |

(continued)

| Strain | Decomposition of $CF_4$ (%, as compared to control group) |
|---|---|
| BL21/pET-SF0757(S184H) | 8.76 |
| BL21/pET-SF0757(S184Q) | 7.35 |
| BL21/pET-SF0757(S184D) | 5.61 |

In Table 6, the control group was *E.* coli BL21 to which an empty pET28a vector was introduced instead of the pET-SF0757 vector, and the wild type was *E. coli* including SF0757.

As shown in Table 6, *E. coli* including the SF0757 wild type gene showed a decrease in the level of $CF_4$ by 3.95% as compared to the control group, and the SF0757 variant, i.e., *E. coli* including the S184H, S184Q, and S184D genes, showed a decrease in the level of $CF_4$ by 8.76%, 7.35%, and 5.61%, respectively, as compared to the control group.

**5. Decomposition of a fluorine-containing compound using a circulation process**

The decomposition rate of the fluorine-containing compound in a sample was measured in the same manner as in section (4) of Example 1, except that strains of the recombinant *E. coli* BL21/pET-SF0757 prepared in section (3) were used.

[Table 7]

| Strain | Decomposition rate of $CF_4$ (%) |
|---|---|
| BL21/pET-SF0757wt | 25.7 |
| BL21/pET-SF0757mt(S184H) | 29.9 |

As shown in Table 7, the SF0757 variant after 144 hours of the culture showed an increase in the degradation rate by 1.16 times the degradation rate of the wild type strain, when applying the gas phase circulation process using the microorganism thereto.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

**1.** A variant haloacid dehalogenase (HAD) superfamily protein, wherein the variant protein comprises an amino acid alteration in at least one amino acid residue corresponding to positions N122 and S184 of SEQ ID NO: 1.

**2.** A polynucleotide encoding the variant haloacid dehalogenase (HAD) superfamily protein of claim 1.

**3.** A recombinant microorganism comprising a foreign gene encoding the variant of a haloacid dehalogenase (HAD) superfamily protein of claim 1.

**4.** The microorganism of claim 3, wherein the amino acid alteration comprises substitution of V or Y for N122 or substitution of a different amino acid for N122 that is conservative with respect to V or Y, wherein the substitution of a different amino acid for N122 that is conservative with respect to V is N122G, N122A, N122L, N122I, N122M, N122F, N122W, or N122P, and the substitution of a different amino acid for N122 that is conservative with respect to Y is N122S, N122T, N122C, or N122Q, or
wherein the amino acid alteration comprises substitution of H, Q, or D for S184 or substitution of a different amino acid for S184 that is conservative with respect to H, Q, or D, wherein the substitution of a different amino acid for S184 that is conservative with respect to H is S184K, or S184R, the substitution of a different amino acid for S184 that is conservative with respect to Q is S184T, S184C, S184Y, or S184N and the substitution of a different amino acid for S184 that is conservative with respect to D is S184E.

**5.** The microorganism of claim 3, wherein the HAD superfamily protein comprises an amino acid sequence having 85% or more sequence identity to SEQ ID NO: 1, 5, 29, 30, 31, 32, or 33, or
wherein the variant HAD superfamily protein comprises an amino acid sequence having 85% or more sequence identity to SEQ ID NO: 1, 5, 29, 30, 31, 32, or 33, and comprises substitution of at least one of N122 and S184 of SEQ ID NO: 1, 5, 29, 30, 31, 32, or 33.

**6.** The microorganism of claim 3, wherein the variant comprises SEQ ID NO: 1 with an N122Y substitution, SEQ ID NO: 5 with an S184H substitution, or SEQ ID NO: 1 or SEQ ID NO: 5 comprising both an N122Y and S184H substitution, or
wherein the variant HAD protein comprises SEQ ID NO: 19, 21, 23, 25, or 27.

**7.** The microorganism of claim 3, wherein the microorganism is *Escherichia.*

**8.** A composition comprising

(a) the variant haloacid dehalogenase (HAD) superfamily protein of claim 1; and
(b) a fluorine-containing compound of Formula 1 or Formula 2:

<Formula 1> $C(R^1)(R^2)(R^3)(R^4)$

<Formula 2> $(R^5)(R^6)(R^7)C\text{-}[C(R^{11})(R^{12})]n\text{-}C(R^8)(R^9)(R^{10})$,

wherein, in Formulae 1 and 2,

n is an integer from 0 to 10;
$R^1$, $R^2$, $R^3$, and $R^4$ are each independently fluorine (F), chlorine (Cl), bromine (Br), iodine (I), or hydrogen (H), wherein at least one of $R^1$, $R^2$, $R^3$, or $R^4$ is F; and

$R^5$, $R^6$, $R^7$, $R^8$, $R^9$, $R^{10}$, $R^{11}$, and $R^{12}$ are each independently F, Cl, Br, I, or H, wherein at least one of $R^5$, $R^6$, $R^7$, $R^8$, $R^9$, $R^{10}$, $R^{11}$, or $R^{12}$ is F.

**9.** The composition of claim 8, wherein the amino acid alteration comprises substitution of V or Y for N122 or substitution of a different amino acid for N122 that is conservative with respect to V or Y, wherein the substitution of a different amino acid for N122 that is conservative with respect to V is N122G, N122A, N122L, N122I, N122M, N122F, N122W, or N122P, and the substitution of a different amino acid for N122 that is conservative with respect to Y is N122S, N122T, N122C, or N122Q, or
wherein the amino acid alteration comprises substitution of H, Q, or D for S184 or substitution of a different amino acid for S184 that is conservative with respect to H, Q, or D, wherein the substitution of a different amino acid for S184 that is conservative with respect to H is S184K, or S184R, the substitution of a different amino acid for S184 that is conservative with respect to Q is S184T, S184C, S184Y, or S184N and the substitution of a different amino acid for S184 that is conservative with respect to D is S184E.

**10.** The composition of claim 8, wherein the composition comprises a recombinant microorganism comprising a foreign gene that expresses the variant, wherein the microorganism preferably belongs to the genus *Escherichia.*

**11.** The composition of claim 8, wherein the variant HAD superfamily protein comprises an amino acid sequence having 85% or more sequence identity to SEQ ID NO: 1, 5, 29, 30, 31, 32, or 33, and comprises substitution in at least one of N122 and S184 of SEQ ID NO: 1, 5, 29, 30, 31, 32, or 33.

**12.** A method of reducing a concentration of a fluorine-containing compound in a sample, the method comprising:

contacting the variant haloacid dehalogenase (HAD) superfamily protein of claim 1 with a sample comprising a fluorine-containing compound represented by Formula 1 or Formula 2, so as to reduce the concentration of the fluorine-containing compound in the sample:

<Formula 1> $C(R_1)(R_2)(R_3)(R_4)$

<Formula 2> $(R_5)(R_6)(R_7)C\text{-}[C(R_{11})(R_{12})]_n\text{-}C(R_8)(R_9)(R_{10})$.

wherein, in Formulae 1 and 2,

n is an integer from 0 to 10;
$R^2$, $R^3$, and $R^4$ are each independently fluorine (F), chlorine (Cl), bromine (Br), iodine (I), or hydrogen (H), wherein at least one of $R^2$, $R^3$, or $R^4$ is F; and
$R^5$, $R^6$, $R^7$, $R^8$, $R^9$, $R^{10}$, $R^{11}$, and $R^{12}$ are each independently F, Cl, Br, I, or H, wherein at least one of $R^5$, $R^6$, $R^7$, $R^8$, $R^9$, $R^{10}$, $R^{11}$, or $R^{12}$ is F.

**13.** The method of claim 12, wherein the amino acid alteration comprises substitution of V or Y for N122 or substitution of a different amino acid for N122 that is conservative with respect to V or Y, wherein the substitution of a different amino acid for N122 that is conservative with respect to V is N122G, N122A, N122L, N122I, N122M, N122F, N122W, or N122P, and the substitution of a different amino acid for N122 that is conservative with respect to Y is N122S, N122T, N122C, or N122Q, or
wherein the amino acid alteration comprises substitution of H, Q, or D for S184 or substitution of a different amino acid for S184 that is conservative with respect to H, Q, or D, wherein the substitution of a different amino acid for S184 that is conservative with respect to H is S184K, or S184R, the substitution of a different amino acid for S184 that is conservative with respect to Q is S184T, S184C, S184Y, or S184N and the substitution of a different amino acid for S184 that is conservative with respect to D is S184E.

**14.** The method of claim 12, wherein the variant HAD superfamily protein comprises an amino acid sequence having 85% or more sequence identity to SEQ ID NO: 1, 5, 29, 30, 31, 32, or 33, and comprises substitution in at least one of N122 and S184 of SEQ ID NO: 1, 5, 29, 30, 31, 32, or 33.

**15.** The method of claim 12, wherein the variant HAD protein is comprised in a recombinant microorganism comprising a foreign gene that expresses the variant HAD protein, wherein contacting the variant HAD protein with the sample comprising the fluorine-containing compound preferably comprises culturing the microorganism with the sample.

FIG. 1

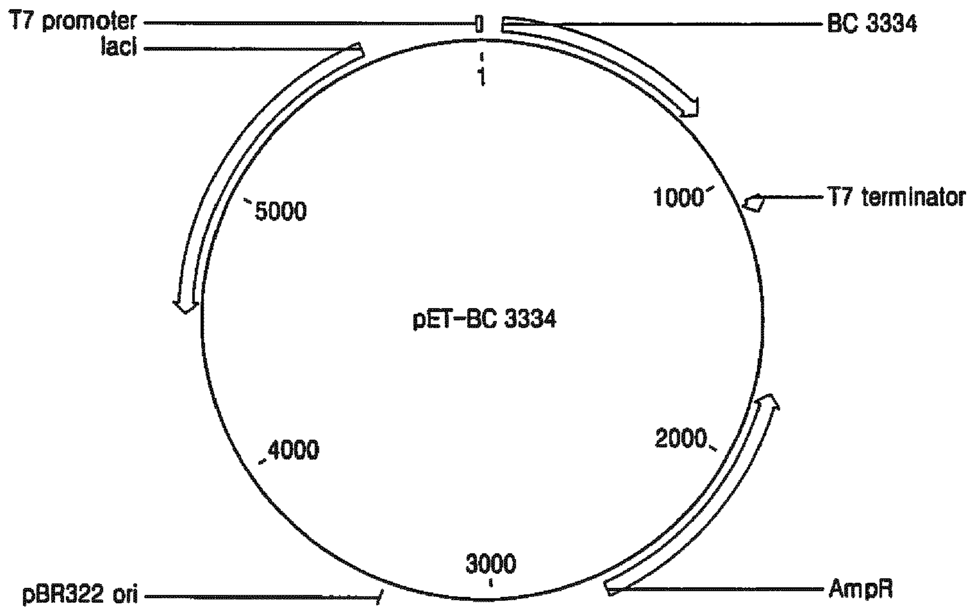
T7 promoter
laci
BC 3334
1
1000
T7 terminator
5000
pET-BC 3334
4000
2000
3000
pBR322 ori
AmpR

FIG. 2

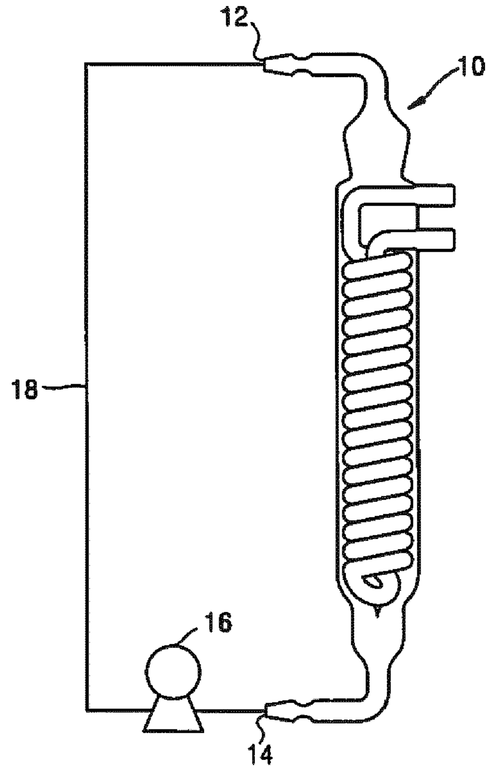

FIG. 3

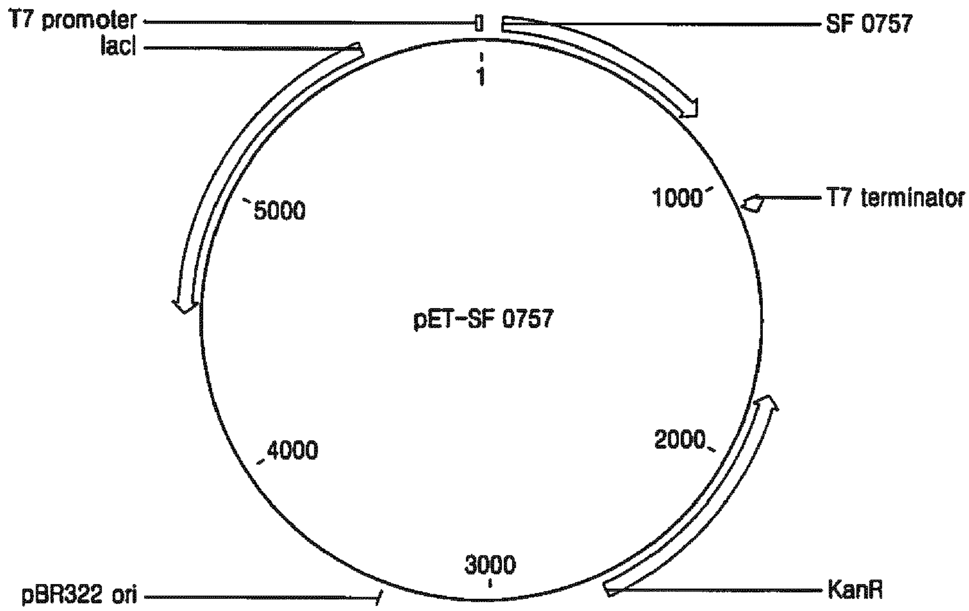
T7 promoter
lacI
SF 0757
1
1000
T7 terminator
5000
pET-SF 0757
4000
2000
3000
pBR322 ori
KanR

FIG. 4

```
btc_CT43_CH3258      MKYKVILFDVDDTLLDFPKTERHALHNAFVQFDHPTGYNDYLASYKEISNGLWRDLENKM
btg_BTB_c33930       MKYKVILFDVDDTLLDFPKTERHALHNAFVQFDHPTGYNDYLASYKEISNGLWRDLENKM
bce_BC3334           MKYKVILFDVDDTLLDFPETERHALHNAFVQFDHPTGYNDYLASYKEISNGLWRDLENKM
btb_BMB171_C3020     MKYKVILFDVDDTLLDFPETERHALHNAFVQFDHPTGYNDYLASYKEISNGLWRDLENKM
bcb_BCB4264_A3346    MKYKVILFDVDDTLLDFPETERHALHNAFVQFGHSTGYNDYLASYKEISNGLWRDLENKM
bti_BTG_02795        MKYKVILFDVDDTLLDFPETERHALHNAFVQFGHPTGYNDYLASYKEISNGLWRDLENKM
                     ******************:*************.*.*************************

btc_CT43_CH3258      ITLSELAVDRFRQLFALHNIDVDAQQFSDVYLENLGKEVHLIEGAVQLCENLQDCKLGII
btg_BTB_c33930       ITLSELAVDRFRQLFALHNIDVDAQQFSDVYLENLGKEVHLIEGAVQLCENLQDCKLGII
bce_BC3334           ITLSELAVDRFRQLFALHNIDVDAQQFSDVYLENLGKEVHLIEGAVQLCENLQDCKLGII
btb_BMB171_C3020     ITLSELAVNRFRQLFALHNIDVDAQQFSDVYLENLGKEVHLIEGAVQLCENLQDCKLGII
bcb_BCB4264_A3346    ITLSELAVNRFRQLFALHNIDVDAQQFSDVYLENLGKEVHLIEGAVQLCENLQDCKLGII
bti_BTG_02795        ITLSELAVDRFRQLFALHNINVDAQQFSDVYLENLGKEVHLIEGAVQLCENLQDCKLGII
                     ********:***********:***************************************

btc_CT43_CH3258      TNGYTKVQQSRIGNSPLSNFFDHIIISEEVGHQKPAREIFDYAFEKFGITDKSSVLMVGD
btg_BTB_c33930       TNGYTKVQQSRIGNSPLSNFFDHIIISEEVGHQKPAREIFDYAFEKFGITDKSSVLMVGD
bce_BC3334           TNGYTKVQQSRIGNSPLCNFFDHIIISEEVGHQKPAREIFDYAFEKFGITDKSSVLMVGD
btb_BMB171_C3020     TNGYTKVQQSRIGNSPLCNFFDHIIISEEVGHQKPAREIFDYAFEKFGITDKSSVLMVGD
bcb_BCB4264_A3346    TNGYTMVQQSRIGNSPLCNFFDHIIISEEVGHQKPAREIFDYAFEKFGITDKSSVLMVGD
bti_BTG_02795        TNGYTKVQQSRIGNSPLCNFFDHIIISEEVGHQKPAREIFDYAFEKFGITDKSSVLMVGD
                     ***** ***********.******************************************

btc_CT43_CH3258      SLTSDMKGGEDYGIDTCWYNPSLKENGTDVNPTYEVESLLQILEIVEVAEEKVASF (SEQ ID NO:29)
btg_BTB_c33930       SLTSDMKGGEDYGIDTCWYNPSLKENGTDVNPTYEVESLLQILEIVEVAEEKVASF (SEQ ID NO:30)
bce_BC3334           SLTSDMKGGEDYGIDTCWYNPSLKENGTDVNPTYEVESLLQILEIVEVAEEKVASF (SEQ ID NO:1)
btb_BMB171_C3020     SLTSDMKGGEDYGIDTCWYNPSLKENGTDVNPTYEVESLLQILEIVEVAEEKVASF (SEQ ID NO:31)
bcb_BCB4264_A3346    SLTSDMKGGEDYSIDTCWYNPSLKENGTDVNPTYEVESLLQILEIVEVAEEKVASF (SEQ ID NO:32)
bti_BTG_02795        SLTSDMKGGEDFGIDTCWYNPSLKENKTSVNPTYEVESLLQILEIVEVAEEKVASF (SEQ ID NO:33)
                     ***********:.************* *.***************************
```

Europäisches Patentamt
European Patent Office
Office européen des brevets

# EUROPEAN SEARCH REPORT

Application Number
EP 18 19 2063

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,P | EP 3 309 246 A1 (SAMSUNG ELECTRONICS CO LTD [KR]) 18 April 2018 (2018-04-18)<br>* whole document esp. paragraphs [1,4] and claims *<br>----- | 1-15 | INV.<br>C12N9/14 |
| Y,P | EP 3 329 981 A2 (SAMSUNG ELECTRONICS CO LTD [KR]) 6 June 2018 (2018-06-06)<br>* whole document esp. paragraph [5] and claim 14 *<br>----- | 1-15 | |
| X | DATABASE UniParc [Online]<br>Uniprot;<br>19 March 2013 (2013-03-19),<br>XP002787322,<br>Database accession no. UPI00026C56D9<br>* sequence *<br>----- | 1 | |
| Y | US 2017/114348 A1 (CHU HUNSU [KR] ET AL) 27 April 2017 (2017-04-27)<br>* whole document esp. paragraph [33] and claim 20 *<br>----- | 1-15 | TECHNICAL FIELDS SEARCHED (IPC) |
| Y | EP 2 374 875 A2 (PROMEGA CORP [US]) 12 October 2011 (2011-10-12)<br>* whole document esp. paragraphs [11,84] *<br>----- | 1-15 | C12N |

The present search report has been drawn up for all claims

1

| Place of search | Date of completion of the search | Examiner |
|---|---|---|
| Munich | 12 December 2018 | Brück, Marianne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 18 19 2063

This annex lists the patent family members relating to the patent documents cited in the above-mentioned European search report. The members are as contained in the European Patent Office EDP file on
The European Patent Office is in no way liable for these particulars which are merely given for the purpose of information.

12-12-2018

| Patent document cited in search report | Publication date | Patent family member(s) | Publication date |
|---|---|---|---|
| EP 3309246 A1 | 18-04-2018 | CN 107955801 A | 24-04-2018 |
| | | EP 3309246 A1 | 18-04-2018 |
| | | KR 20180042016 A | 25-04-2018 |
| | | US 2018104647 A1 | 19-04-2018 |
| EP 3329981 A2 | 06-06-2018 | CN 108144434 A | 12-06-2018 |
| | | EP 3329981 A2 | 06-06-2018 |
| | | KR 20180063752 A | 12-06-2018 |
| | | US 2018154308 A1 | 07-06-2018 |
| US 2017114348 A1 | 27-04-2017 | NONE | |
| EP 2374875 A2 | 12-10-2011 | CA 2667697 A1 | 08-05-2008 |
| | | EP 2087107 A2 | 12-08-2009 |
| | | EP 2374875 A2 | 12-10-2011 |
| | | EP 2492342 A1 | 29-08-2012 |
| | | EP 2502990 A2 | 26-09-2012 |
| | | JP 5680302 B2 | 04-03-2015 |
| | | JP 5840117 B2 | 06-01-2016 |
| | | JP 2010508023 A | 18-03-2010 |
| | | JP 2013078329 A | 02-05-2013 |
| | | US 2008145882 A1 | 19-06-2008 |
| | | US 2013302880 A1 | 14-11-2013 |
| | | US 2015140636 A1 | 21-05-2015 |
| | | US 2017137793 A1 | 18-05-2017 |
| | | US 2018223267 A1 | 09-08-2018 |
| | | WO 2008054821 A2 | 08-05-2008 |

EPO FORM P0459

For more details about this annex : see Official Journal of the European Patent Office, No. 12/82